# EUROPÄISCHE PATENTANMELDUNG

(11) **EP 3 482 743 A1**
(43) Veröffentlichungstag der Anmeldung: **15.05.2019**
(21) Anmeldenummer: 17201650.3
(22) Anmeldetag: 14.11.2017
(51) Int. Cl.: A61K 9/00, A61K 45/06, A61K 47/10, A61K 47/18, A61K 9/06, A61K 9/107, A61K 31/192, A61K 31/196, A61K 31/4174, A61P 31/04, A61P 31/10, A61P 29/00, A61P 15/02

(54) **EMULSIONEN ZUR BEHANDLUNG VON SCHEIDENINFEKTIONEN**

(71) Anmelder: ProFem GmbH, 1180 Wien (AT)
(72) Erfinder: NOE, Marion, 1180 Wien (AT); NOE, Christian, 1180 Wien (AT)
(74) Vertreter: Sonn & Partner Patentanwälte

(57) **Zusammenfassung**

Die Erfindung betrifft eine Emulsion, zB in Form eines Salbe oder einer Creme, mit einer wässerigen Phase und einer Ölphase, enthaltend ein Antimykotikum und ein NSAID, dadurch gekennzeichnet, dass (a) das NSAID in der wässerigen Phase in einem Konzentrationsbereich vorliegt, welcher der Hälfte bis einem Zehntel der für diese Wirkstoffe üblichen Konzentration entspricht, dass (b) das Gewichtsverhältnis der Wasser- zur Ölphase in dieser Emulsion zwischen den Werten 1,5 und 2,7 liegt, und dass (c) der pH Wert der Emulsion nicht unter dem Wert 6,5 und nicht über 8,5 vorzugsweise im Bereich 7,0 bis 8 liegt, insbesondere zur Anwendung in der topischen Behandlung von vaginalen Pilzinfektionen.

## Beschreibung

Die WO 2007/131253 A2 betrifft die Verwendung von einem antimykotischen Wirkstoff und einem Epithelzellen- oder Endothelzellen-Adhäsionsinhibitor zur Herstellung eines Kombinationsarzneimittels zur topischen Behandlung von Candidamykosen ausgewählt aus vulvovaginaler Candidiasis, oropharyngealer Candidiasis (Mundsoor), Windeldermatitis (Windelsoor) und intertriginösem Ekzem.

Die vorliegende Erfindung stellt sich zur Aufgabe, verbesserte Kombinationspräparate aus einem Antimykotikum und einem NSAID zur Verfügung zu stellen, die besonders gut geeignet zur topischen Behandlung von vaginalen und dermalen Pilzinfektionen sind. Derartige Emulsionen sollen auch bei hartnäckigen Pilzinfektionen, welche besonders im Vaginalbereich auftreten, wirksam sein, sowie mikrobiologisch und chemisch stabil sein.

Demgemäß betrifft die vorliegende Erfindung eine Emulsion, vorzugsweise in Form einer Salbe oder einer Creme, welche eine wässerige Phase und eine Ölphase (Fettphase) umfasst, enthaltend ein Antimykotikum und ein NSAID, dadurch gekennzeichnet, dass (a) das NSAID in der wässerigen Phase in einem Konzentrationsbereich vorliegt, welcher der Hälfte bis einem Zehntel der für diese Wirkstoffe in anderen Formulierungen üblichen Konzentration in Bezug auf die Einzeldosis entspricht, dass (b) das Gewichtsverhältnis der wässerigen Phase zur Ölphase in dieser Emulsion zwischen 2,2 bis 2,8 liegt, und dass (c) der pH Wert der Emulsion nicht unter dem Wert 6,5 und nicht über 8,5 vorzugsweise im Bereich 7,0 bis 8 liegt, vorzugsweise zur Behandlung von dermalen und vaginalen Pilzinfektionen, insbesondere zur Anwendung in der topischen Behandlung von vaginalen Pilzinfektionen.

Die vorliegende Erfindung stellt verbesserte Präparate der eingangs beschriebenen Art zur Verfügung. Insbesondere zeigen die erfindungsgemäßen Präparate neben der optimierten Pharmakokinetik durch den Angriff unmittelbar am Infektionsort auch eine optimale Pharmakodynamik. Damit wird nicht nur eine besonders gute Wirksamkeit des Antimykotikums ermöglicht, sondern auch das Zusammenwirken mit dem NSAID signifikant verbessert, indem durch eine niedrige Konzentration an NSAID trotzdem eine ausreichende Wirksamkeit gesichert wird, ohne die dabei auftretenden Nebenwirkungen (Reizungen, Brennen, etc.) in Kauf nehmen zu müssen. In der Praxis führt dies dazu, dass zuvor aus diesen Nebenwirkungsgründen nicht einsetzbare Kombinationen aus Antimykotikum und NSAID mit der Lehre der vorliegenden Erfindung den Patientinnen zugänglich gemacht werden können und diese Patientinnen nunmehr einer erfolgreichen Therapie zugeführt werden können. Erfindungsgemäß hat sich aber gezeigt, dass für eine optimale pharmakodynamische Wirkung die Konzentration des NSAID in der wässerigen Phase von entscheidender Bedeutung ist. Die Verfügbarmachung des eingebrachten NSAIDs wird durch das Zusammenwirken des Herstellungsprozesses, des Wasser/Öl-Verhältnisses und des pH-Wertes, durch welchen das überwiegende Vorliegen des NSAIDs in seiner Salzform gewährleistet wird, erreicht.

Die bevorzugten NSAIDs, die erfindungsgemäß zum Einsatz kommen, sind ausgewählt aus der Gruppe, bestehend aus Diclofenac, Bufexamac, Ibuprofen, Dexibuprofen, Ketoprofen, Flurbiprofen, Piroxicam, Meloxicam, Flufenaminsäure Mefenaminsäure, Naproxen und Indometacin. Die übliche Konzentration, in der Diclofenac dermal topisch eingesetzt wird, liegt im Bereich von 1 bis 2% (10 mg/bzw. 20 mg/g). Erfindungsgemäß wird Diclofenac eingesetzt in einer Menge von 0,1% bis 0,5 % (1 - 5 mg pro g Creme), vorzugsweise von 0,2.%. bis 0,4%. Die übliche Konzentration, in der Ibuprofen topisch eingesetzt wird, liegt bei 5% (50 mg/g Creme/Gel). Erfindungsgemäß wird Ibuprofen eingesetzt in einer bevorzugten Menge von 0,5 bis 2,5% (5 - 25 mg pro g Creme), vorzugsweise von 1 bis 2%. Weitere Beispiele für bevorzugte NSAIDs und deren bevorzugte Mengen ("Erfindungsgemäße Konzentration") können aus Tabelle A ersehen werden.

**Tabelle A:**

| Wirkstoff | Einzeldosis | Tageshöchstdosis | Beispiel | Einzeldosis | Tageshöchstdosis | Beispiel | **Erfindungsgemäße Konzentration** |
|---|---|---|---|---|---|---|---|
| | oral/i.v. | | | topisch | | | |
| Diclofenac Natrium | 50 - 75 mg | 150 - 225 mg | Voltaren 50 mg Tabletten | | | Voltadol Schmerzgel 1%, 10 mg/g | **0,2 - 0,5 Gew%** |
| | | | 75 mg Amp. | 40 - 80 mg | 240 mg | 2%, 20 mg/g | |
| Indometacin | 25 - 75 mg | 50 - 150 mg | Indocid 25 mg | 20-40 mg | 40-80 mg | IndometGel | **0,1 - 0,4 Gew.%** |
| | | | 75 mg ret. | | | 1%, 10 mg/g | |
| Naproxen | 250 - 500 mg | 1000 mg | Naproxen FT 250/500 mg | | | | |
| | | | Naproxen Susp. 50 mg/ml | | | | **0,5** - **2,5 Gew%** |
| Ibuprofen | 200 - 800 mg | 2400 mg | Ibuprofen FT 200/ 400/800 mg | | | Ibutop Creme/ Gel | |
| | | | | 100-250 mg | 1000 mg | 5%, 5g/100 g | **0,5-2,5 Gew.%** |

Es hat sich erfindungsgemäß gezeigt, dass die Behandlung von Pilzerkrankungen, insbesondere von Candidamykosen, besonders effektiv mit Hilfe eines Kombinationspräparates aus einem Antimykotikum mit einem Arzneistoff erfolgen kann, welcher zugleich auf die Adhäsion der Mikroorganismen Einfluss nimmt. Die Arzneimittel werden vorteilhaft topisch appliziert, weil dadurch der Wirkort unmittelbar erreicht wird und zugleich eine minimale systemische Belastung erfolgt. Solche Wirkstoffkombinationen sind in den Patenten, die aus der WO 2007/131253 A2 abgeleitet sind, geschützt.

Gegenstand der Erfindung sind speziell für die vaginale Applikation besonders geeignete Medikamentenkombinationen, sowie ihre Herstellung und Verwendung. Durch Beachtung der besonderen physiologischen und pathophysiologischen Gegebenheiten in der Scheide erweisen sich sowohl eine spezielle Zusammensetzung als auch ein spezieller Herstellungsprozess als besonders vorteilhaft. Die Beachtung spezifischer Parameter führt dazu, dass bei der Therapie von vaginalen Scheideninfektionen ein besonders guter therapeutischer Effekt erreicht wird.

Bezüglich der adhäsionshemmenden Komponente betrifft die vorliegende Verbindung nur die Gruppe der NSAIDs (non-steroidal antiinflammatoric drugs), weil Arzneistoffe dieser Gruppe neben der antiadhäsiven Wirkung, welche die Anhaftung verhindern, auch eine schmerzlindernde und entzündungshemmende Wirkung haben. Beide Effekte sind vor allem bei chronischen Formen von Candidamykosen sehr willkommen, weil diese mit Entzündung und starken Schmerzen verbunden sind.

Bevorzugte NSAIDs sind dabei Diclofenac, Ibuprofen, Dexibuprofen Ketoprofen, Flubiprofen, Mefenaminsäure, Naproxen und Indometacin. Bevorzugte Antimykotika sind Nystatin, Ciclopirox oder Ciclopiroxolamin, oder Antimykotika aus der Gruppe der Azole (Imidazole, Triazole, Tetraazole) wie Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, VT-1161, SCY-078.

### Strukturformel von SCY-078

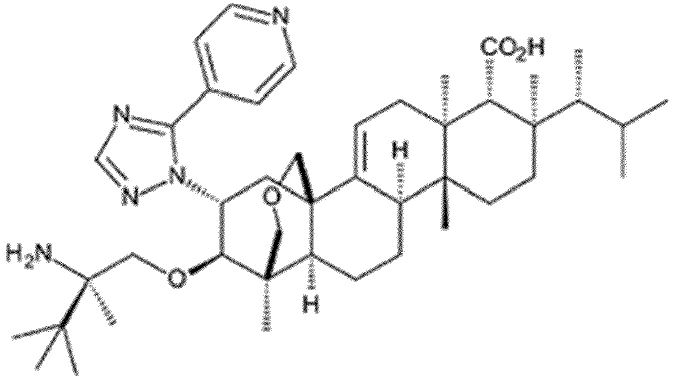

### Strukturformel von VT-1161

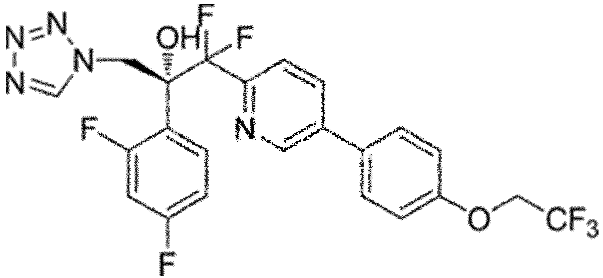

Überraschender Weise hat sich bei der Anwendung gezeigt, dass zur Erzielung einer optimalen Wirkung dem Verhältnis der Anteile des Antimykotikums und des NSAIDs eine besondere Bedeutung zukommt. In den Kombinationen eines Antimykotikums mit einem NSAID kann das Antimykotikum in üblichen Dosierungen eingesetzt werden (Clotrimazol 1-10%, Ketokonazol 1-5%, bevorzugt 2%, Nystatin 100.000 IE/ml bzw g). Da das NSAID seine Wirkung in gleicher Weise am Erreger in der Scheide und am Scheidenepithel ausübt, ist zum einen die topische Applikation des Arzneimittels, bei welcher es ja direkt am Wirkort aufgebracht wird, deutlich einer systemischen Anwendung überlegen, zum anderen sollte deshalb das NSAID in weit niedrigeren Dosen zum Einsatz kommen, als bei den üblichen systemischen Anwendungen als Schmerzmittel. Bei der vaginalen Anwendung wird ein therapeutischer Effekt bereits ab einem Zehntel, vorzugsweise einem Fünftel einer Wirkstoffkonzentration erreicht, welche bei üblichen dermalen Formulierungen von NSAID zur Anwendung kommen (im Falle von Diclofenac 200 bis 500 mg/100g Salbe). Die niedrige Wirkstoffkonzentration ist auch deshalb besonders vorteilhaft, weil die systemische Aufnahme des NSAID vernachlässigbar wird und keinerlei Gefahr systemischer Nebenwirkungen besteht. Zugleich muss beachtet werden, dass es bei der topischen Applikation auf Schleimhäuten leicht zur Überdosierung des NSAID kommen kann (z.B. ist eine Creme mit Diclofenac: ab 0,75 % schmerzhaft; vor der Anwendung am Markt befindlicher NSAID-hältiger Cremes (1-2% Diclofenac, 5% Ibuprofen) auf Schleimhäuten wird in den jeweiligen Gebrauchsinformationen gewarnt, z.B. Gebrauchsinformationen Voltaren Emulgel, Ibutop-Gel), weil es zur Reizung der Schleimhäute kommt, welche dem antiinflammatorischen Effekt entgegenwirkt. Es sollte daher erfindungsgemäß das NSAID in keiner größeren Menge als 50% der üblichen niedrigsten Formulierung für dermale Anwendungen eingesetzt werden. Bei dieser Konzentration können bereits Reizungen auftreten. Damit bewegt sich die einzusetzende Wirkstoffkonzentration der NSAID vorzugsweise in einem relativ kleinen therapeutischen Fenster von 10 - 60, vorzugsweise von 20 - 40% des für topische Formulierungen üblichen Wertes. Demgemäß enthält eine besonders bevorzugte Emulsion gemäß der vorliegenden Erfindung Diclofenac als NSAID, wobei Diclofenac in einem Konzentrationsbereich von 0,2 - 0,4 Gewichtsprozent der Emulsion enthalten ist.

Emulsionen (Öl in Wasser oder Wasser in Öl), vorzugsweise in Form einer Creme, sind besonders gut geeignet, um die Wirkstoffe effizient und konzentriert an die mit Biofilm behafteten Oberflächen heranzubringen. Die Viskosität der Emulsionen wird stark durch das Wasser zu Öl Verhältnis bestimmt. Überraschender Weise zeigte sich, dass bei der Formulierung der Emulsion dem Verhältnis Wasser: Öl eine besondere Bedeutung zukommt. Bei höherem Anteil der Fettbestandteile wird die Entfaltung der Wirksamkeit behindert. Hingegen kommt es bei einem niedrigeren Fettanteil zu einer stärkeren Reizwirkung. Zur Erzielung eines optimalen therapeutischen Effektes soll das Wasser: Öl Verhältnis den Wert von 2,8 nicht übersteigen. Über diesem Bereich wird der Wirkstoff zu schnell mit dem Scheidensekret ausgeschwemmt, wodurch keine ausreichende Zeit gegeben ist, um die zur Adhäsionsunterbindung beitragende Wirkung auf die äußere Schicht des Vaginalepithels, an welchem der Pilz haftet, zu entfalten. Der bei zu hohem Wasser: Öl Verhältnis sehr schnell ausgeschwemmte Wirkstoff kann allenfalls als Nebenwirkung zusätzlich auch einen reizenden Effekt verursachen.

In gleicher Weise, wie ein zu hoher Wasseranteil in der Emulsion einen negativen Effekt ausübt, ist auch ein zu hoher Fettanteil zu vermeiden. In Anbetracht der niedrigen Konzentration, in welcher das NSAID eingesetzt wird, ist es zur Erzielung des therapeutischen Effektes von besonderer Wichtigkeit, dass die Freisetzung des Wirkstoffes am Wirkort rasch und nicht protrahiert erfolgt. Bei einer langsamen Freisetzung, wie sie aus der öligen Phase erfolgt, ist nicht gewährleistet, dass die erforderliche therapeutische Konzentration am Wirkort erreicht wird. Aus diesem Grund soll das Wasser: Öl Gewichtsverhältnis in der Emulsion den Wert von 2,2 nicht unterschreiten. Somit ergibt sich auch für den Wert des Wasser-Öl Gewichtsverhältnisses der erfindungsgemäßen Emulsionen ein Fenster zwischen 2,1 und 2,9, vorzugsweise zwischen 2,2 und 2,8.

Es ist wesentlich, dass die NSAIDs gemäß der Erfindung in Salzform (bzw. ionischer Form) vorliegen und zwar sowohl bei der Einarbeitung als auch bei der Anwendung. Deshalb ist deren Einbringung in die Formulierung von Bedeutung. Bei einer Einarbeitung des NSAID in seiner freien Form oder bei der Einarbeitung als Salz in die ölige Phase wird der therapeutische Effekt massiv beeinträchtigt. Während das Antimykotikum vorzugsweise in die ölige Phase eingearbeitet wird und in dieser vorliegt, wird daher nach dem Verfahren gemäß der Erfindung das NSAID vor Herstellung der Emulsion in der Regel in die wässrige Phase eingebracht. Alternativ dazu kann das feste Salz des NSAID in feinkristalliner oder mikronisierter Form, bzw. als Hydrogel in die (weitgehend) fertige Emulsion eingearbeitet werden.

Eine zügige Freisetzung ist dann gewährleistet, wenn in der Emulsion der Wirkstoff in der wässrigen Phase vorliegt, was voraussetzt, dass das NSAID als Salz vorliegt. Die meisten NSAIDs sind schwache Säuren mit einem pKa-Wert von 4 - 5 (Diclofenac 4,15, Ibuprofen 4,91, Mefenaminsäure 4,2, Indometacin 4,5, Naproxen 4,2). Demgemäß liegen sie bereits im schwach sauren Milieu zum Teil in freier Form vor und werden so in die Ölphase extrahiert, was zu einer reduzierten Wirkung oder zum Wirkungsverlust führen kann. Da mit sinkendem pH-Wert der Wirkstoffanteil des als freie Säure vorliegenden NSAID in der Ölphase zunimmt, ist ein pH-Wert von mindestens pH = 6 zweckmäßig, um bei den Medikamenten in den Wirkstoffkonzentrationen gemäß der Erfindung einen therapeutischen Effekt zu haben.

Die Kombination eines lipophilen Antimykotikums, vom Strukturtyp des Clotrimazol, mit den schwachen Säuren der NSAIDs führt auch dazu, dass die Kombination nur in einem relativ engen pH-Fenster stabil ist. Unter einem pH von 6 wird Clotrimazol instabil, während es bei NSAIDs zu einer Abnahme der chemischen Stabilität im basischen Milieu kommen kann (im Fall von Diclofenac ab pH 8,00 - 8,5). Ebenso muss beachtet werden, dass der pH-Wert einer Emulsion die grundsätzliche physiologische Verträglichkeit beeinflusst. Deshalb sind auch bezüglich des pH-Wertes der Formulierung relativ enge Grenzen gesetzt. Gemäß der Erfindung hat die (wässerige Phase der Emulsion) einen pH-Wert im Bereich von 6,5 bis 8,5, vorzugsweise von 7 - 8.

Da durch die Einbringung des NSAID-Salzes in ein antimykotisches Medikament der pH-Wert alkalischer wird, ist das in vergleichbaren Emulsionen, welche nur Clotrimazol enthalten, üblicherweise verwendete Konservierungsmittel nicht immer ausreichend wirksam. Die halbfesten antimykotischen Medikamente gemäß der vorliegenden Erfindung werden daher vorzugsweise mit solchen Konservierungsmitteln und Antiseptika gegen mikrobiellen Befall geschützt, welche im pH-Bereich der Emulsion wirksam sind. In erfindungsgemäßen Medikamenten, welche zur Behandlung einer reinen Pilzinfektion eingesetzt werden, wird der Gehalt des Antiseptikums zweckmäßiger Weise niedrig gehalten, um die normale Scheidenflora nicht zu beeinträchtigen. In den Biofilmen, welche sich durch das Wachstum der Pilze bilden, sind häufig nicht nur Pilze, sondern auch andere pathogene Mikroorganismen enthalten. Alleine schon durch eine entsprechende Erhöhung der Konzentration des Antiseptikums können auch solche Mischinfektionen therapeutisch erfasst werden. Deshalb ist die Zugabe von einer höheren, für eine akute antimikrobielle Wirkung ausreichenden Menge eines Antiseptikums zu den beanspruchten Medikamenten ein weiterer Gegenstand der Erfindung. Bevorzugte Antiseptika sind quartäre Ammoniumsalze, wie Benzalkoniumchlorid, und Dequaliniumchlorid, sowie Phenoxyethanol und Propylenglykol.

Unter den bakteriellen Mikroorganismen, welche sich in den vaginalen Biofilmen finden, spielen typische Darmkeime, wie Enterobacter, E. coli und/oder etwa Klebsiella pneumoniae sowie Anaerobier wie Gardnerella vaginalis und Prevotella spp. eine besondere Rolle. Diese werden von den beanspruchten Antiseptika wohl ebenfalls erfasst, es ist jedoch zweckmäßig, in nachgewiesenen Fällen einer derartigen Infektion, an Stelle des Antiseptikums oder ergänzend zu diesem ein gegen Anaerobier aktives Antibiotikum einzusetzen. Deshalb ist die Zugabe eines Antibiotikums, welches gegen anaerobe Keime wirkt, ein weiterer Gegenstand der Erfindung. Bevorzugte Antibiotika sind Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin und Cotrimoxazol.

Entscheidend für das Aufbrechen des Biofilms ist die Anwesenheit eines NSAID in den beschriebenen Konzentrationen und Bedingungen in einer Emulsion in der beschriebenen Zusammensetzung und in den beschriebenen Mengenverhältnissen.

Schleimhautoberflächen bieten ideale Voraussetzungen für die Ausbildung von Biofilmen, welche besonders therapieresistent sind. Das vaginale Mikrobiom trägt durch die Beeinflussung des feuchten, physiologisch sauren Milieus der Scheide entscheidend zur Wirksamkeit topisch applizierter Arzneiformen bei, da die Verfügbarkeit der Arzneistoffe pH-abhängig ist. Diese Bedingungen sind eine der Mitursachen, dass die derzeit vermarkteten topischen Antimykotika nur einen sehr eingeschränkten Erfolg bei der Therapie chronischen Vulvovaginitis, vor allem bei Vorliegen von Mischinfektionen mit Bakterien, haben. Die Zusammensetzung der erfindungsgemäßen Arzneistoffkombinationen ist in den beschriebenen Kombinationen für die Behandlung auch komplexer chronischer Scheidenentzündungen ganz besonders geeignet.

Da es auch bei sehr vielen dermalen Pilzinfektionen zur Ausbildung von Biofilmen kommt, sind Emulsionen gemäß dieser Erfindung auch sehr gute zur Behandlung von Mykosen, insbesondere Candidamykosen, aber auch Infektionen mit Malassezia geeignet.

Demgemäß dienen die erfindungsgemäßen Emulsionen vorzugsweise zur Anwendung in der Behandlung von dermalen und vaginalen Pilzinfektionen, insbesondere zur Anwendung in der topischen Behandlung von vaginalen Pilzinfektionen. Generell können die erfindungsgemäßen Emulsionen zur Anwendung in der Behandlung von Infektionskrankheiten herangezogen werden, insbesondere von vaginalen Infektionen durch Candida albicans oder von vaginalen Mischinfektionen durch Candida albicans und Bakterien, wie Enterobacter, E.coli, Klebsiella pneumoniae, Gardnerella vaginalis, Prevotella spp. Eine besondere Ausführungsform der vorliegenden Erfindung liegt in der Anwendung in der Behandlung bakterieller Vaginosen. Eine weitere besondere Ausführungsform der vorliegenden Erfindung liegt in der Anwendung in der Behandlung dermaler Pilzinfektionen vorzugsweise von Candidamykosen, insbesondere vulvovaginale Candidiasis, oropharyngeale Candidiasis (Mundsoor), Windeldermatitis (Windelsoor), und intertriginöses Ekzem, und Malassziamykosen (Pityriasis versicolor).

Die Erfindung wird anhand der nachfolgenden Beispiele näher erläutert, auf die sie jedoch nicht eingeschränkt ist.

### Beispiele:

### Vorbemerkung

Veränderungen im Herstellungsverfahren und in der prozentuellen Zusammensetzung eines Öl-Wassergemisches entsprechend Basisrezeptur A zeigten überraschende Veränderungen der klinischen Wirksamkeit abseits von üblichen Dosis-Wirkungsbeziehungen. Ableitbar ist die deutlich verbesserte oder ggf. auch verringerte Wirksamkeit aus einem besonders raschen Wirkungseintritt (lokale Schmerzstillung) bzw. einer Verzögerung des Wirkungseintrittes oder der Verstärkung vorbestehender Schmerzen.

### Beispiel 1 - Basisrezeptur

**Tab.1: Basisrezeptur A**

| Zusammensetzung der Emulsion | |
|---|---|
| Clotrimazol | 1,00 |
| Diclofenac Na | 0,20 |
| Sorbitanmonostearat | 2,00 |
| Polysorbat 60 | 1,50 |
| Cetylpalmitat | 3,00 |
| 2-Octyldodecanol | 13,50 |
| Cetostearylalkohol | 10,00 |
| Benzylaalkohol | 1,00 |
| Gereinigtes Wasser Ph.Eu. | 67,80 |
| Gesamt | 100,00 |
| Ph | 7,8 |

Bei Konzentrationsänderungen des NSAIDs es wird dieses jeweils gegen gereinigtes Wasser ausgetauscht, der Gehalt der Lipidbestandteile bleibt, wenn nicht anders angeführt, gleich. Die klinische Wirksamkeit in den folgenden angeführten Präparationen wird in Bezug zu einer klinisch gut wirksamen Basisrezeptur A gesetzt.

### Beispiel 2: Variationen des pH-Bereiches und klinische Wirksamkeit

Veränderungen in Bezug auf die Konservierungsmittel sind mit Veränderungen des pH-Wertes verbunden. Die Herstellung der genannten Beispiele erfolgt nach allgemeiner Arbeitsvorschrift 1. Die Einstellung des pH-Wertes zur Erreichung des Wirkungsoptimums des jeweiligen Konservierungsmittels erfolgt durch Zugabe geeigneter Pufferlösungen, durch welche die jeweilige Menge an gereinigtem Wasser ersetzt wurde.

Der pH-Wert hat über die Verschiebung des freien Wirkstoffanteils gegenüber dem als Salz vorliegenden Anteil einen wesentlichen Einfluss auf die lokal bioverfügbare Wirkstoffmenge. In Abhängigkeit von den pKa-Werten der eingesetzten nicht-steroidalen Antiphlogistika ergibt sich daraus ein pH-Optimum der erfindungsgemäßen Kombinationspräparate gemäß der vorliegenden Erfindung.

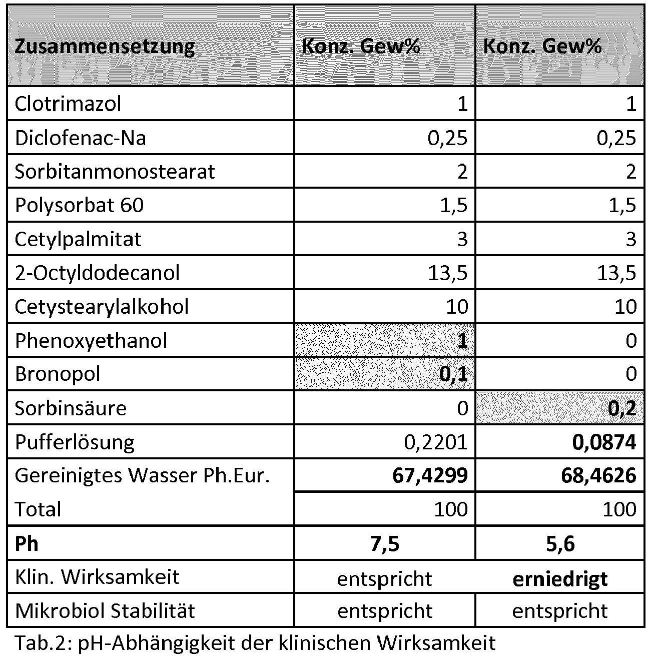

### Beispiel 3: Einfluss des Gewichtsverhältnisses wässerige Phase/Öl

Veränderungen der Viskosität zeigen überraschenderweise schon bei geringer Variationsbreite deutliche Einflüsse auf die klinische Wirksamkeit. Die Herstellung der genannten Beispiele erfolgt nach allgemeiner Arbeitsvorschrift 1 durch Variationen im Gehalt der fettigen Bestandteile und des Wassergehaltes.

Eine Erhöhung des Wasseranteils und damit Erniedrigung der Viskosität führt über eine verstärkte Freisetzung und stärkere Benetzung der Schleimhäute zu lokalen Reizerscheinungen und verminderter klinischer Wirksamkeit.

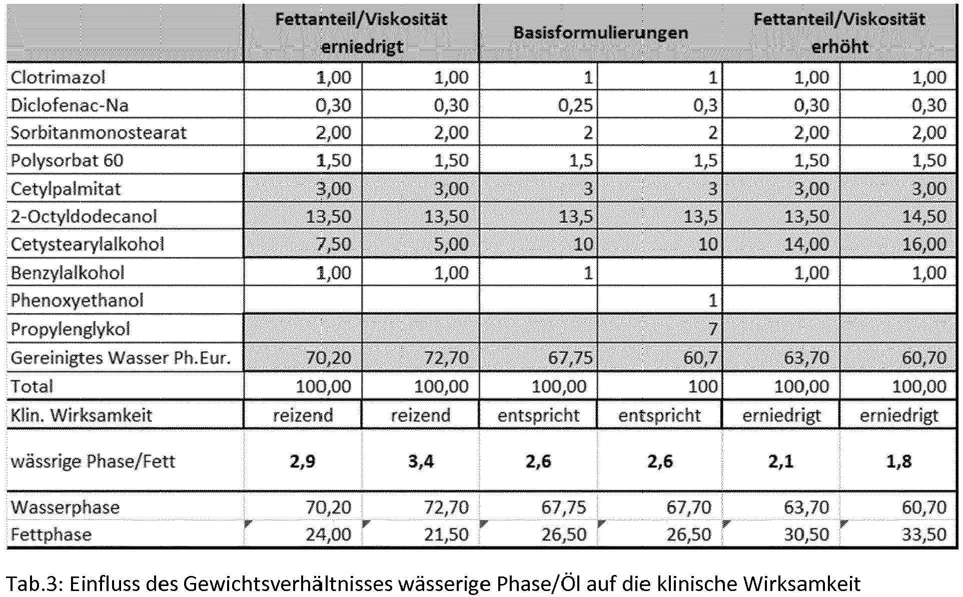

### Beispiel 4: Variation des nicht-steroidalen Antiphlogistikums

Zu Basisrezeptur A wurden anstelle von Diclofenac verschiedene andere nicht-steroidale Antiphlogistika beigemengt und auf ihre klinische Wirksamkeit untersucht.

**Tab.4: Variationen von nicht-steroidalen Antiphlogistika**

| Formu lierung Nr. | Nicht-steroidiales Antiphlogistikum | Präparation | Herstellungsvariante | Wirksamkeit |
|---|---|---|---|---|
| 1.1 | Mefenaminsäure | 1 g/100 g | Basisrezeptur A + Mefenaminsäure mikronisiert | entspricht |
| 1.2 | Indometacin | 0,15 g/100 g | Basisrezeptur A + Indometacin mikronisiert | entspricht |
| 1.3 | Ibuprofen | 1 g/100 g | Basisrezeptur A + Ibuprofen in Hydrogel | entspricht |
| 1.4 | Ibuprofen | 0,5 g/100 g | Basisrezeptur A + Ibuprofen in Hydrogel | entspricht |
| 1.5. | Naproxen | 1 g/100 g | Basisrezeptur A + Naproxen mikronisiert | entspricht |

### Beispiel 5: Konzentrationsoptimum

Gemäß Basisrezeptur A wurden Emulsionen mit verschiedenen Konzentrationen von Diclofenac Na hergestellt und auf ihre klinische Wirksamkeit untersucht.

**Tab.5: Abhängigkeit der klinischen Wirksamkeit von der Konzentration des NSAIDs**

| | **Konz. Gew%** | **Konz. Gew%** | **Konz. Gew%** | **Konz. Gew%** |
|---|---|---|---|---|
| Clotrimazol | 1 | 1 | 1 | 1 |
| Diclofenac-Na | 0,1 | 0,25 | 0,5 | 0,75 |
| Sorbitanmonostearat | 2 | 2 | 2 | 2 |
| Polysorbat 60 | 1,5 | 1,5 | 1,5 | 1,5 |
| Cetylpalmitat | 3 | 3 | 3 | 3 |
| 2-Octyldodecanol | 13,5 | 13,5 | 13,5 | 13,5 |
| Cetystearylalkohol | 10 | 10 | 10 | 10 |
| Benzylalkohol | 1 | 1 | 1 | 1 |
| Gereinigtes Wasser Ph.Eur. | 67,9 | 67,75 | 67,5 | 67,25 |
| Total | 100 | 100 | 100 | 100 |
| **Ph** | **7,6** | **7,8** | **8,1** | **8,1** |
| Klin. Wirksamkeit | leicht erniedrigt | entspricht | entspricht | schleimhautreizend |

### Beispiel 6: Variation von Konservierungsmitteln und mikrobiologische Stabilität

Durch den gemeinsamen Einsatz von Clotrimazol und NSAIDs verändern sich durch die pH-Verschiebungen im System der Emulsion sowohl mikrobiologische als auch die chemische Stabilität [Lit. Pharmakopöe] gegenüber einer vergleichbaren Clotrimazolformulierung.

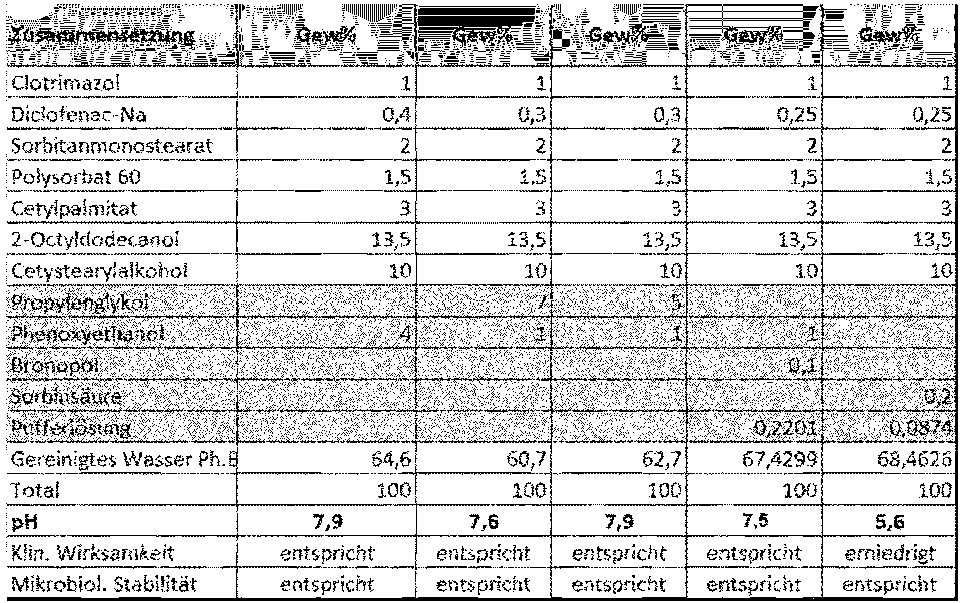

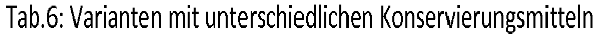

### Beispiel 7:

### Herstellung von Basisrezeptur A,

### Allgemeine Herstellungsvorschrift 1:

Die Komponenten Sorbitanmonostearat, Polysorbat 60, Cetylpalmitat, 2-Octyldodecanol und Cetostearylalkohol werden bei einer Temperatur von 70-75°C aufgeschmolzen. In die Klarschmelze werden unter Rühren bei einer Temperatur von 60 °C - 70 °C Clotrimazol und anschließend Benzylalkohol zugegeben. Parallel hierzu wird Diclofenac Natrium in gereinigtem Wasser unter Erwärmung aufgelöst. Die wässrige Lösung wird unter Rühren zur Ölphase zugegeben und homogenisiert. Unter langsamem Abkühlen unter weiterer Homogenisierung der entstehenden w/o-Emulsion findet eine Phasenumkehr statt, bei welcher eine hydrophile, homogene Creme entsteht.

**Tab. 8: Formulierungen nach Basis Rezeptur A, Herstellungsvorschrift 1**

| Bestandteile der Emulsion | | |
|---|---|---|
| Clotrimazol | 1,00 | 1,00 |
| Diclofenac Na | 0,20 | 0,30 |
| Sorbitanmonostearat | 2,00 | 2,00 |
| Polysorbat 60 | 1,50 | 1,50 |
| Cetylpalmitat | 3,00 | 3,00 |
| 2-Octyldodecanol | 13,50 | 13,50 |
| Cetostearylalkohol | 10,00 | 10,00 |
| Benzylalkohol | 1,00 | 1,00 |
| Gereinigtes Wasser Ph.Eu. | 67,80 | 67,70 |
| | 100,00 | 100,00 |

Die klinische Wirksamkeit der Rezepturen in Tabelle 8 ist ident.

### Allgemeine Herstellungsvorschrift 2

Die Komponenten Sorbitanmonostearat, Polysorbat 60, Cetylpalmitat, 2-Octyldodecanol und Cetostearylalkohol werden bei einer Temperatur von 70-75°C aufgeschmolzen. In die Klarschmelze wird unter Rühren bei einer Temperatur von 60 °C - 70 °C Clotrimazol zugegeben und aufgeschmolzen. Parallel hierzu wird Diclofenac Natrium in gereinigtem Wasser unter Erwärmung aufgelöst. Nach Zugabe von Phenoxyethanol und gegebenenfalls Propylenglykol wird die wässrige Lösung bei einer Temperatur von 60°C - 70°C C unter Rühren zur Ölphase zugegeben und homogenisiert. Unter langsamem Abkühlen unter weiterer Homogenisierung der anfangs entstehenden w/o-Emulsion findet eine Phasenumkehr zu einer o/w-Emulsion statt, bei welcher eine hydrophile, homogene Creme entsteht. (Klin. Ergebnisse siehe Tab.6)

Die Einarbeitung des NSAIDs kann anstelle des Auflösens in Wasser auch durch Einrühren eines in einem Hydrogel aufgelösten NSAID oder durch Einrühren des (mikronisierten) NSAIDs als Feststoff während der Emulsionsbereitung erfolgen.

### Beispiel 9: Herstellung von Basisrezeptur A durch Einarbeitung des NSAID in die Ölphase

Cremes mit verschiedenen Konzentrationen von Diclofenac Na wurden nach Basisrezeptur A nach allgemeiner Herstellungsvorschrift 2 hergestellt und auf ihre klinische Wirksamkeit untersucht. Gereinigtes Wasser wurde gegen Diclofenac Na ausgetauscht, der Gehalt der Lipidbestandteile, des O/W-Emulgators Polysorbat 60 und des Konservierungsmittels Benzylalkohol war in den Rezepturen identisch.

### Allgemeine Herstellungsvorschrift 3

Die Komponenten Sorbitanmonostearat, Polysorbat 60, Cetylpalmitat, 2-Octyldodecanol und Cetostearylalkohol werden aufgeschmolzen. In die Klarschmelze wird unter Rühren Benzylalkohol gegeben (Lipidphase). Gereinigtes Wasser wird zum Sieden erhitzt und 70% der erforderlichen Masse entsprechend Rezeptur unter Rühren in die Lipidphase gegeben (Phasenumkehr und Bildung einer O/W-Präemulsion). Etwa 30% der Präemulsion werden aus dem Kessel genommen und darin Clotrimazol und Diclofenac-Natrium dispergiert. Die wirkstoffhaltige Präemulsion wird in den Kessel zur wirkstofffreien Präemulsion gegeben und mit gereinigtem Wasser (restliche 30%) auf die Endmasse aufgefüllt. Die Creme wird bis zum Erreichen von Raumtemperatur gerührt und abschließend zur Homogenisierung zweimal über den Dreiwalzenstuhl gegeben (pH 6,99).

| Zusammensetzung | | |
|---|---|---|
| Clotrimazol | 2,00 | 2,00 |
| Diclofenac Na | 0,10 | 0,20 |
| Sorbitanmonostearat | 2,00 | 2,00 |
| Polysorbat 60 | 1,50 | 1,50 |
| Cetylpalmitat | 3,00 | 3,00 |
| 2-Octyldodecanol | 13,50 | 13,50 |
| Cetostearylalkohol | 10,00 | 10,00 |
| Benzylaalkohol | 1,00 | 1,00 |
| Gereinigtes Wasser Ph.Eu. | 66,90 | 66,80 |
| | 100,00 | 100,00 |
| Klin. Wirksamkeit | erniedrigt | erniedrigt |

Aus den Beispielen nach Herstellungsvorschrift 3 ergibt sich ein wesentlicher Einfluss des Herstellungsprozesses, der so gestaltet sein muss, dass das nicht-steroidale Antiphlogistikum über die wässrige Phase eingearbeitet wird und vorwiegend in der wässrigen Phase vorliegt.

### Beispiel 10: Herstellung von Basisrezeptur A

Herstellung von Basisrezeptur A mit Einarbeitung eines nicht-steroidialen Antiphlogistikums (Diclofenac Na) nach allgemeiner Herstellungsvorschrift 4:
Die Komponenten Sorbitanmonostearat, Polysorbat 60, Cetylpalmitat, 2-Octyldodecanol und Cetostearylalkohol werden aufgeschmolzen. In die Klarschmelze wird unter Rühren Benzylalkohol gegeben (Lipidphase). Gereinigtes Wasser wird zum Sieden erhitzt und 70% der erforderlichen Masse entsprechend Rezeptur unter Rühren in die Lipidphase gegeben (Phasenumkehr und Bildung einer O/W-Präemulsion). Etwa 30% der Präemulsion werden aus dem Kessel genommen und darin Clotrimazol dispergiert. Die wirkstoffhaltige Präemulsion wird in den Kessel zur wirkstofffreien Präemulsion gegeben und mit gereinigtem Wasser (restliche 30%) auf die Endmasse aufgefüllt. Die Creme wird unter Zugabe von mikronisiertem Diclofenac Natrium in mehreren Portionen bis zum Erreichen der Raumtemperatur gerührt und abschließend zur Homogenisierung zweimal über den Dreiwalzenstuhl gegeben.

Demgemäß betrifft die Erfindung die nachfolgenden, bevorzugten Ausführungsformen:
1. Eine Emulsion mit einer wässerigen Phase und einer Ölphase, enthaltend ein Antimykotikum und ein NSAID, dadurch gekennzeichnet, dass (a) das NSAID in der wässerigen Phase in einem Konzentrationsbereich vorliegt, welcher der Hälfte bis einem Zehntel der für diese Wirkstoffe üblichen Konzentration entspricht, dass (b) das Gewichtsverhältnis der Wasser- zur Ölphase in dieser Emulsion zwischen den Werten 2,1 und 2,9 liegt, und dass (c) der pH Wert der Emulsion nicht unter dem Wert 6,5 und nicht über 8,5 vorzugsweise im Bereich 7,0 bis 8 liegt.
2. Eine Emulsion gemäß Ausführungsform 1 dadurch gekennzeichnet, dass sie in Form einer Salbe oder einer Creme vorliegt.
3. Eine Emulsion gemäß Ausführungsform 1 oder 2, dadurch gekennzeichnet, dass das Antimykotikum Nystatin, Ciclopirox oder Ciclopiroxolamin, oder ein Antimykotikum aus der Gruppe der Azole, vorzugsweise Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, VT-1161 oder SCY-078, ist.
4. Eine Emulsion gemäß einer der Ausführungsformen 1 bis 3, dadurch gekennzeichnet, dass das NSAID Diclofenac, vorzugsweise in einer Konzentration von 0,2 bis 0,5 Gewichtsprozent, Ibuprofen, vorzugsweise in einer Konzentration von 0,5 bis 2,5 Gewichtsprozent, Bufexamac, Dexibuprofen, Flurbiprofen, Ketoprofen, Piroxicam, Meloxicam, Flufenaminsäure, Mefenaminsäure, Indometacin, vorzugsweise in einer Konzentration von 0,1 bis 0,4 Gewichtsprozent, oder Naproxen, vorzugsweise in einer Konzentration von 0,5 bis 2,5 Gewichtsprozent, ist.
5. Eine Emulsion gemäß einer der Ausführungsformen 1 bis 4, dadurch gekennzeichnet, dass das NSAID Diclofenac ist und dieses in einem Konzentrationsbereich von 0,2 - 0,4 Gewichtsprozent der Emulsion enthalten ist.
6. Eine Emulsion gemäß einer der Ausführungsformen 1 bis 5, in welcher ein im pH-Bereich der Emulsion aktives Konservierungsmittel enthalten ist.
7. Eine Emulsion gemäß Ausführungsform 6, in welcher das Konservierungsmittel Phenoxyethanol oder Propylenglykol oder eine Kombination dieser beiden ist.
8. Eine Emulsion gemäß Ausführungsform 6, in welcher das Konservierungsmittel Dequaliniumchlorid ist.
9. Eine Emulsion gemäß einer der Ausführungsformen 1 bis 8, dadurch gekennzeichnet, dass weiters ein gegen bakterielle Keime wirkendes Antibiotikum enthalten ist.
10. Eine Emulsion gemäß Ausführungsform 9, dadurch gekennzeichnet, dass das Antibiotikum Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, oder Cotrimoxazol ist.
11. Eine Emulsion gemäß einer der Ausführungsformen 1 bis 10 zur Anwendung in der Behandlung von dermalen und vaginalen Pilzinfektionen.
12. Eine Emulsion gemäß einer der Ausführungsformen 1 bis 11 zur Anwendung in der topischen Behandlung von vaginalen Pilzinfektionen.
13. Emulsion gemäß einer der Ausführungsform 1 bis 12 zur Anwendung in der Behandlung von Infektionskrankheiten, insbesondere von vaginalen Infektionen durch Candida albicans.
14. Emulsion gemäß einer der Ausführungsformen 1 bis 13 zur Anwendung in der Behandlung von Infektionskrankheiten, insbesondere von vaginalen Mischinfektionen durch Candida albicans und Bakterien, wie Enterobacter, E.coli, Klebsiella pneumoniae, Gardnerella vaginalis, Prevotella spp.
15. Emulsion gemäß einer der Ausführungsformen 1 bis 14 zur Anwendung in der Behandlung bakterieller Vaginosen.
16. Emulsion gemäß den Ausführungsformen 1 bis 15 zur Anwendung in der Behandlung dermaler Pilzinfektionen vorzugsweise von Candidamykosen, insbesondere vulvovaginale Candidiasis, oropharyngeale Candidiasis (Mundsoor), Windeldermatitis (Windelsoor), und intertriginöses Ekzem, und Malassziamykosen.
17. Ein Verfahren zur Herstellung einer Emulsion gemäß einer der Ausführungsformen 1 bis 16, dadurch gekennzeichnet, dass bei der Herstellung der Emulsion das NSAID über die wässrige Phase eingebracht wird,
18. Ein Verfahren zur Herstellung einer Emulsion gemäß einer der Ausführungsformen 1 bis 16, dadurch gekennzeichnet, dass das NSAID als feinkristallines oder mikronisiertes Salz in die das Antimykotikum enthaltene Emulsion eingebracht wird.
19. Ein Verfahren zur Herstellung einer Emulsion gemäß einer der Ausführungsformen 1 bis 16, dadurch gekennzeichnet, dass das NSAID über ein Hydrogel in die das Antimykotikum enthaltene Emulsion eingebracht wird.
20. Ein Verfahren zur Herstellung einer Emulsion gemäß einer der Ausführungsformen 1 bis 16, bei welchem die Zugabe der Stoffe so erfolgt, dass eine therapeutisch wirksame antibakterielle Wirkung erzielt wird.

## Patentansprüche

1. Eine Emulsion mit einer wässerigen Phase und einer Ölphase, enthaltend ein Antimykotikum und ein NSAID, **dadurch gekennzeichnet, dass** (a) das NSAID in der wässerigen Phase in einem Konzentrationsbereich vorliegt, welcher der Hälfte bis einem Zehntel der für diese Wirkstoffe üblichen Konzentration entspricht, dass (b) das Gewichtsverhältnis der Wasser- zur Ölphase in dieser Emulsion zwischen den Werten 2,1 und 2,9 liegt, und dass (c) der pH Wert der Emulsion nicht unter dem Wert 6,5 und nicht über 8,5 vorzugsweise im Bereich 7,0 bis 8 liegt.

2. Eine Emulsion gemäß Anspruch 1 **dadurch gekennzeichnet, dass** sie in Form einer Salbe oder einer Creme vorliegt.

3. Eine Emulsion gemäß Anspruch 1 oder 2, **dadurch gekennzeichnet, dass** das Antimykotikum Nystatin, Ciclopirox oder Ciclopiroxolamin, oder ein Antimykotikum aus der Gruppe der Azole, vorzugsweise Clotrimazol, Fluconazol, Miconazol, Itraconazol, Tioconazol, Voriconazol, Bifonazol, Econazol, Isoconazol, Fenticonazol, Sertaconazol, Ketoconazol, Posaconazol, Quilseconazol, VT-1161 oder SCY-078, ist; und/oder dass das NSAID Diclofenac, vorzugsweise in einer Konzentration von 0,2 bis 0,5 Gewichtsprozent, Ibuprofen, vorzugsweise in einer Konzentration von 0,5 bis 2,5 Gewichtsprozent, Bufexamac, Dexibuprofen, Flurbiprofen, Ketoprofen, Piroxicam, Meloxicam, Flufenaminsäure, Mefenaminsäure, Indometacin, vorzugsweise in einer Konzentration von 0,1 bis 0,4 Gewichtsprozent, oder Naproxen, vorzugsweise in einer Konzentration von 0,5 bis 2,5 Gewichtsprozent, ist.

4. Eine Emulsion gemäß einem der Ansprüche 1 bis 3, **dadurch gekennzeichnet, dass** das NSAID Diclofenac ist und dieses in einem Konzentrationsbereich von 0,2 - 0,4 Gewichtsprozent der Emulsion enthalten ist.

5. Eine Emulsion gemäß einem der Ansprüche 1 bis 4, in welcher ein im pH-Bereich der Emulsion aktives Konservierungsmittel enthalten ist.

6. Eine Emulsion gemäß Anspruch 5, in welcher das Konservierungsmittel Phenoxyethanol oder Propylenglykol oder eine Kombination dieser beiden ist.

7. Eine Emulsion gemäß Anspruch 5, in welcher das Konservierungsmittel Dequaliniumchlorid ist.

8. Eine Emulsion gemäß einem der Ansprüche 1 bis 7, **dadurch gekennzeichnet, dass** weiters ein gegen bakterielle Keime wirkendes Antibiotikum enthalten ist, wobei das das Antibiotikum vorzugsweise Phosphomycin, Clindamycin, Metronidazol, Nitrofurantoin, Nitrofurazon, Nitrofurantoin, Nifuratel, Nifuroxacin, Nitroxolin, Trimethoprim, Sulfadiazin, oder Cotrimoxazol ist.

9. Eine Emulsion gemäß einem der Ansprüche 1 bis 8 zur Anwendung in der Behandlung von dermalen und vaginalen Pilzinfektionen.

10. Eine Emulsion gemäß einem der Ansprüche 1 bis 8 zur Anwendung in der topischen Behandlung von vaginalen Pilzinfektionen.

11. Emulsion gemäß einem der Anspruch 1 bis 10 zur Anwendung in der Behandlung von Infektionskrankheiten, insbesondere von vaginalen Infektionen durch Candida albicans; zur Anwendung in der Behandlung von vaginalen Mischinfektionen durch Candida albicans und Bakterien, wie Enterobacter, E.coli, Klebsiella pneumoniae, Gardnerella vaginalis, Prevotella spp.; Emulsion gemäß einem der Ansprüche 1 bis 14 zur Anwendung in der Behandlung bakterieller Vaginosen; und/oder zur Anwendung in der Behandlung dermaler Pilzinfektionen vorzugsweise von Candidamykosen, insbesondere vulvovaginale Candidiasis, oropharyngeale Candidiasis (Mundsoor), Windeldermatitis (Windelsoor), und intertriginöses Ekzem, und Malassziamykosen.

12. Ein Verfahren zur Herstellung einer Emulsion gemäß einem der Ansprüche 1 bis 11, **dadurch gekennzeichnet, dass** bei der Herstellung der Emulsion das NSAID über die wässrige Phase eingebracht wird,

13. Ein Verfahren zur Herstellung einer Emulsion gemäß einem der Ansprüche 1 bis 11, **dadurch gekennzeichnet, dass** das NSAID als feinkristallines oder mikronisiertes Salz in die das Antimykotikum enthaltene Emulsion eingebracht wird.

14. Ein Verfahren zur Herstellung einer Emulsion gemäß einem der Ansprüche 1 bis 11, **dadurch gekennzeichnet, dass** das NSAID über ein Hydrogel in die das Antimykotikum enthaltene Emulsion eingebracht wird.

15. Ein Verfahren zur Herstellung einer Emulsion gemäß einem der Ansprüche 1 bis 11, bei welchem die Zugabe der Stoffe so erfolgt, dass eine therapeutisch wirksame antibakterielle Wirkung erzielt wird.
